(19) Europäisches Patentamt
European Patent Office
Office européen des brevets

(11) Numéro de publication: **0 208 576**
**A1**

(12) # DEMANDE DE BREVET EUROPEEN

(21) Numéro de dépôt: 86401227.3

(22) Date de dépôt: 06.06.86

(51) Int. Cl.$^4$: A 61 K 47/00
A 61 K 31/765

(30) Priorité: 07.06.85 FR 8508648

(43) Date de publication de la demande: 14.01.87 Bulletin 87/3

(84) Etats contractants désignés: AT BE CH DE FR GB IT LI LU NL SE

(71) Demandeur: CENTRE NATIONAL DE LA RECHERCHE SCIENTIFIQUE (CNRS)
15, Quai Anatole France
F-75007 Paris(FR)

(72) Inventeur: Delmotte, Francis
150, rue Philippe de Commynes
F-45160 Olivet(FR)

(72) Inventeur: Monsigny, Michel
341, rue des Bouvreuils
F-45590 Saint Cyr en Val(FR)

(72) Inventeur: Lescanne, Pierre Jean
85, rue des Ruelles Brières les Scellés
F-91150 Etampes(FR)

(72) Inventeur: Daussin, François
39, rue des Anguiguin bât. Les Rouches
F-45650 Saint Jean Le Blanc(FR)

(74) Mandataire: Nony, Michel
Cabinet NONY & CIE 29, rue Cambacérès
F-75008 Paris(FR)

(54) **Nouvelles prodrogues macromoléculaires hydrosolubles, leur préparation et leur utilisation comme médicaments, notamment antitumoraux et antiparasitaires.**

(57) Utilisation dans la réalisation d'une prodogue, d'un polymère polyhydroxylé auquel la drogue est reliée par l'intermédiaire d'un groupement peptidyle, et prodrogues ainsi obtenues.

Application à l'obtention de médicaments notament antitumoraux et antiparasitaires.

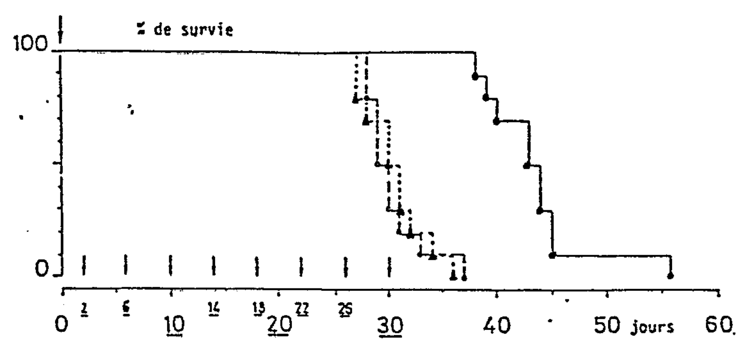


Figure 5 : Mesure de la survie des animaux traités ; Jo : implantation intramusculaire de $10^5$ cellules 3 LL, ↓ : jours d'injection des différents produits, o—o—o : sérum physiologique, ▲-▲-▲ : Daunorubicine libre (100 microgrammes injection, ●--●--● : Polymère de l'exemple 2 (200 microgrammes/injection en équivalents de Daunorubicine).

EP 0 208 576 A1

Croydon Printing Company Ltd.

Nouvelles prodrogues macromoléculaires hydrosolubles, leur préparation et leur utilisation comme médicaments, notamment antitumoraux et antiparasitaires.

La présente invention a pour objet de nouvelles prodrogues macromoléculaires hydrosolubles, leur préparation et leur utilisation comme médicament.

On sait que l'on peut obtenir des prodrogues, permettant de conférer notamment un effet retard ou de faciliter le transport de la drogue *in vivo*, en liant la drogue, c'est-à-dire le principe actif, à un peptide, mais la faible solubilité dans l'eau de ces derniers constitue souvent un handicap pour la préparation et l'efficacité de ces prodrogues.

La présente invention a pour objet de nouvelles prodrogues caractérisées par le fait qu'elles comprennent essentiellement une drogue ou substance active liée par une liaison covalente à un polymère polyhydroxylé par l'intermédiaire d'un groupement peptidyle, ledit polymère polyhydroxylé étant un polymère dont au moins 20% des motifs sont des motifs polyhydroxylés et en particulier celles caractérisées par le fait que le rapport du nombre moyen de molécules de substance active fixée sur une molécule de polymère au nombre moyen de groupements hydroxyle présents sur une molécule du polymère peut varier de $10^{-2}$ à $10^{-4}$ environ.

Autrement dit, les prodrogues macromoléculaires de la présente demande répondent à la formule I :

$$M\text{-}(P\text{-}D)_n \qquad (I)$$

dans laquelle M est le reste d'une molécule polymérique dont au moins 20% des motifs sont des motifs polyhydroxylés, P est un groupement peptidyle, D est le reste de la molécule de drogue et n représente le nombre de molécules de drogue D fixées sur la molécule polymérique M.

De préférence, la drogue est une drogue aminée liée au groupement peptidique P par l'extrémité C-terminale de celui-ci, et dans ce cas le peptide P est lié à la molécule polymérique M par l'intermédiaire d'une liaison covalente (par exemple une liaison amide) ; cette liaison covalente provient de la réaction de l'extrémité N-terminale du peptide avec un groupement réactif (par exemple un groupement carboxylique) porté par la molécule polymérique (correspondant à M) utilisée dans la préparation de la prodrogue.

Parmi les composés de formule I on citera en particulier :

A) ceux pour lesquels M représente le reste d'un polymère d'acrylamide substitué à l'azote par un groupement hydrocarboné polyhydroxylé.

Parmi les polymères de ce type on citera notamment ceux qui sont décrits dans le brevet français n° 77.23223 (n° de publication 2.398.762) ;

B) ceux pour lesquels M est le reste d'un homopolymère d'aminoacide diaminé dont le groupement aminé non impliqué dans la liaison polymérique est substitué par un groupement polyhydroxyalcanoyle ;

Parmi les dérivés de ce type on citera en particulier les molécules polymériques à base de polylysine dont les groupements $-NH_2$ non impliqués dans la liaison polymérique sont acylés par un agent d'acylation dérivé d'un acide polyhydroxyalcanoïque ;

C) ceux pour lesquels M est le reste d'un homopolymère d'acide aminé dicarboxylique dont une partie des groupements acide carboxylique non impliqués dans la liaison polymérique sont amidifiés par une polyhydroxyalkylamine dont l'amine est primaire ou secondaire.

Dans ce cas, les groupements carboxyliques restants de la molécule correspondant à M permettent d'établir les liaisons covalentes avec les groupements -(P-D) indiqués dans la formule I. Parmi les dérivés de ce type, on mentionnera en particulier les dérivés d'acide polyglutamique ou d'acide polyaspartique.

Autrement dit, les polymères de formule I sont en particulier :

a) les polymères IA constitués essentiellement de motifs de formules:

$$-CH(R^1)-C(R^2)(X^1)- \quad \text{et} \quad -CH(R^1)-C(R^2)(X^2)-$$

(IIA) (IIIA)

dans lesquelles :

$R_1$ et $R_2$ représentent -H ou un groupement alkyle inférieur,

$X^1$ représente un groupement $-CO-NH-R^3$,

$X^2$ représente un groupement -CO-NH-X-CO-P-D,

$R^3$ est un groupement hydrocarboné polyhydroxylé

X représente un groupement hydrocarboné ayant 1 à 8 atomes de carbone, éventuellement interrompu par 1 ou plusieurs hétéroatomes d'oxygène, de soufre ou d'azote, et P et D sont définis comme précédement ;

b) les polymères IB constitués essentiellement de motifs de formules:

$$-CO-CH(X^3)-NH- \quad \text{et} \quad -CO-CH(X^4)-NH-$$

(IIB) (IIIB)

dans lesquelles :

$X^3$ représente $-(CH_2)_x-NH-CO-R^4$,

$X^4$ représente $-(CH_2)_x-NH-CO-P-D$,

x est un nombre entier pouvant varier de 1 à 4, $R_4$ est un groupement hydrocarboné polyhydroxylé, et P et D sont définis comme précédemment ;

c) les polymères IC constitués essentiellement de motifs de formules :

$-CO-CH(X^5)-NH-$ et $-CO-CH(X^6)-NH-$

(IIC) (IIIC)

dans lesquelles :

$X^5$ représente $-(CH_2)_y-CO-NH-R^5$,

$X^6$ représente $-(CH_2)_y-CO-P-D$,

y est un nombre entier pouvant varier de 1 à 4

$R_5$ est un groupement hydrocarboné polyhydroxylé,

et P et D sont définis comme précédemment.

Bien entendu, l'invention s'étend à l'utilisation de copolymères contenant, outre les motifs mentionnés ci-dessus, des motifs dérivés de co-monomères permettant de faire varier, de façon connue, les propriétés du polymère. Comme indiqué précédemment, les polymères IA, IB et IC contiennent respectivement au moins 20% de motifs IIA, IIB et IIC.

Généralement, la masse moléculaire, déterminée par exemple par la méthode de diffusion de la lumière du polymère correspondant à M, est supérieure à 5000. Elle est comprise par exemple entre 10 000 et 500 000.

Parmi les peptides représentés par P, on citera en particulier ceux qui comprennent une séquence peptidique hydrolysable par une protéase spécifique, en particulier une protéase sécrétée par les cellules cibles de la drogue active, par exemple par les cellules tumorales, par un micro-organisme pathogène, ou par des cellules associées à une tumeur, ou encore après endocytose, par les protéase des lysosomes. L'intérêt de l'utilisation de telles séquences peptidiques est que, dans ce cas, la drogue active n'est libérée que dans l'environnement de la cible ou, après endocytose, à l'intérieur même des cellules cibles, ce qui a pour effet de favoriser l'efficacité de la drogue.

D'une façon générale, les séquences peptidiques représentées par p doivent être considérées comme connues.

Parmi les dérivés de formule I, on citera en particulier ceux pour lesquels les groupements -P-D sont ceux qui comportent les séquences énumérées ci-après (sans que cette liste soit limitative) étant entendu que les séquences indiquées sont les séquences minimales et qu'il est possible d'insérer 1 à 3 autres acides aminés entre la molécule M et la séquence peptidique indiquée :

-L-Ala-L-Ala-L-Pro-L-Ala-D

-L-Ala-L-Ala-L-Pro-L-Ala-L-Leu-D

-L-Ala-L-Ala-L-Pro-L-Ala-D

-L-Ala-L-Ala-L-Pro-L-Ala-L-Leu-D

-L-Ala-L-Ala-L-Pro-L-Val-D

-L-Ala-L-Ala-L-Pro-L-Val-L-Leu-D

-L-Ala-L-Ala-L-Pro-L-Val-D

-L-Ala-L-Ala-L-Pro-L-Val-L-Leu-D

-L-Phé-L-Val-L-Arg-D

-L-Phé-L-Val-L-Arg-L-Leu-D

-L-Phé-L-Val-L-Arg-D

-L-Phé-L-Val-L-Arg-L-Leu-D

-L-Phé-L-Ala-L-Lys-D

-L-Phé-L-Ala-L-Lys-L-Leu-D

-L-Phé-L-Ala-L-Lys-D

-L-Phé-L-Ala-L-Lys-L-Leu-D

-Gly-Gly-L-Arg-D

-Gly-Gly-L-Arg-L-Leu-D

-Gly-Gly-L-Arg-D

-Gly-Gly-L-Arg-L-Leu-D

-Gly-Gly-L-Tyr-D

-Gly-Gly-L-Tyr-L-Leu-D

-Gly-Gly-L-Tyr-D

-Gly-L-Phé-L-Ala-L-Leu-D

-Gly-L-Pro-L-Arg-L-Leu-D

-L-Pro-L-Phé-L-Arg-D

-L-Pro-L-Phé-L-Arg-L-Leu-D

-L-Pro-L-Phé-L-Arg-D

-L-Pro-L-Phé-L-Arg-L-Leu-D

-L-Val-L-Leu-Gly-L-Arg-D

La drogue D est par exemple la daunorubicine, l'adriamycine, la puromycine, qui sont des drogues antitumorales ; la primaquine et la chloroquine qui sont des drogues antiparasitaires, etc...

L'invention concerne également l'utilisation, dans la réalisation d'une prodrogue, d'un polymère polyhydroxylé tel que défini ci-dessus auquel la drogue est reliée par l'intermédiaire d'un groupement peptidyle.

Le polymère et le bras peptidyle sont notamment ceux qui sont définis ci-dessus.

L'invention a également pour objet un procédé de préparation des prodrogues macromoléculaires définies ci-dessus.

Ce procédé est principalement caractérisé par le fait :

a) soit que l'on utilise comme produit de départ une macromolécule contenant des motifs polyhydroxylés que l'on éthérifie avec un groupement hydrocarboné comportant un groupement carboxylique protégé, que l'on soumet l'éther obtenu à une réaction de déprotection du groupe carboxylique et que l'on fait agir le composé obtenu avec un composé peptidyl-drogue tel qu'un composé de formule :

$$H - P - D$$

dans laquelle P et D sont définis comme précédemment ;

b) soit que l'on utilise comme produit de départ une macromolécule polycarboxylée dont le groupement carboxylique est activé et que l'on fait réagir lesdits groupements carboxyliques activés, partiellement avec un composé peptidyl-drogue tel qu'un composé de formule :

$$H - P - D$$

P étant défini comme précédemment, et partiellement avec une amine primaire ou secondaire polyhydroxylée telle qu'une amine de formule :

$$R_5-NH_2$$

$R_5$ étant défini comme précédemment ;

c) soit que l'on utilise comme produit de départ une macromolécule polyaminée, que l'on fait réagir avec un agent d'acylation dérivé d'un acide carboxylique polyhydroxylé pour obtenir, par réaction d'amidification, une molécule polyhydroxylée sur laquelle on poursuit la synthèse comme en a) ci-dessus.

Dans des modes d'exécution particuliers, le procédé de l'invention peut encore présenter les caractéristiques suivantes, prises isolément ou en combinaison :

<u>Procédé a)</u> :

- On éthérifie la macromolécule polyhydroxylée par formation d'un alcoolate alcalin et réaction de celui-ci avec un dérivé halogéné du groupement hydrocarboné comportant un groupement carboxylique protégé ;

- Le groupement carboxylique est protégé par exemple sous forme d'un ester, en particulier d'un ester d'alkyle inférieur et, dans ce cas, le groupement carboxylique est déprotégé par saponification ;

- On fait réagir le groupement carboxylique déprotégé avec le dérivé peptidique H-P-D en présence d'un agent d'activation ; l'agent d'activation est par exemple un carbodiamide tel que le dicyclohexyl carbodiamide en présence d'hydroxybenzotriazole ou tout autre agent d'activation classique dans la synthèse peptidique.

Procédé b) :

- Le groupement carboxylique de la macromolécule polycarboxylée est activé dans les conditions analogues à celles utilisées dans le procédé a.

Procédé c) :

- L'agent d'acylation avec lequel on fait réagir la macromolécule polyaminée est par exemple une lactone telle que la glucono-delta-lactone, la ribono-lactone, la lactone des acides heptaglyconoïque ou octaglyconoïque, etc...

Les produits de formule : H - P - D

sont obtenus par couplage de la séquence peptidique P, protégée du côté N-terminal selon les méthodes connues (par exemple par formation du dérivé N-tritylé) sur la drogue D présentant une fonction aminée, en présence d'un agent d'activation tel que par exemple l'hydroxybenzotriazole, puis déprotection du groupement N-terminal. Les conditions de déprotection de ce produit de couplage doivent évidemment être compatibles avec la stabilité de la drogue.

Les peptides de départ, correspondant à la séquence P dans la formule I, peuvent être soit achetés dans le commerce, soit synthétisés selon les méthodes classiques de la synthèse peptidique (voir par exemple E. CROSS et J. MEIENHOFFER (1979) "The peptides : Analysis, synthesis, biology" Academic Press, New York, Volumes I, II, III et suivants).

L'invention a également pour objet des compositions pharmaceutiques caractérisées par le fait qu'elles renferment, comme ingrédient actif, au moins une prodrogue I, IA, IB ou IC, telle que définie ci-dessus, éventuellement en association avec un excipient approprié.

L'invention a également pour objet l'utilisation des prodrogues telles que définies ci-dessus, dans la préparation industrielle de médicaments.

Les prodrogues de l'invention sont utilisées comme médicaments à des doses correspondant aux doses usuelles de la drogue active qu'elles renferment, ou même à des doses supérieures, car l'expérience a montré que ces prodrogues ont une toxicité inférieure à celle d'une quantité équivalente de la drogue qu'elles renferment.

Les prodrogues de l'invention ont, bien entendu, les mêmes propriétés thérapeutiques que les drogues qu'elles renferment.

Les drogues présentes dans les prodrogues de l'invention sont notamment des agents antitumoraux, des agents antibactériens, ou des agents antiparasitaires.

Lorsque ces drogues sont des agents tumoraux, les médicaments de l'invention sont utilisés notamment comme agents d'inhition de la prolifération des tumeurs ou comme agents de prévention de la formation de métastases. Les doses efficaces varient généralement de 1 à 20 mg/kg de principe actif.

Lorsque les drogues présentes dans les médicaments de l'invention sont des agents antiparasitaires, ces médicaments sont utilisés notamment comme agents de traitement ou de prévention dans le domaine de la lutte contre les parasites responsables des diverses maladies dites "tropicales".

Les médicaments de l'invention sont administrés par toutes voies convenables. Il faut noter toutefois que la voie orale ne convient généralement pas.

Il peuvent être présentés notamment sous forme de solutions injectables, de poudres lyophilisées à diluer dans le sérum physiologique, de solutions ou d'émulsions à appliquer sur la peau ou les muqueuses, de solutions en conditionnement pressurisé pour aérosols, etc...

Les exemples suivants illustrent l'invention sans toutefois la limiter.

Exemple 1 :

Préparation d'un polymère linéaire de N-acryloylamino-2-hydroxyméthyl-2-propanediol-1,3-polycarboxylé substitué par la séquence -Gly-Gly-L-Tyr-Daunorubicine.

Stade 1

A un polymère linéaire de N-acryloyl-amino-2-hydroxyméthyl-2-propanediol-1,3 de masse moléculaire moyenne 200 000 (1g ; 5 micromoles) obtenu selon le procédé du brevet français 77.23223 (2.398.762) en solution dans le N,N-diméthylformamide anhydre (20ml), on ajoute de l'hydrure de sodium (0,8 mmoles). Après 12 heures d'agitation à température ambiante, on additionne à la solution visqueuse de polyalcoolate du méthylbromoacétate (0,4 mmoles). La réaction est maintenue sous agitation 4 heures à température ambiante. Le mélange réactionnel est ensuite neutralisé par une résine de marque commerciale Dowex AG-50 W X 8 (4 ml) puis dialysé contre de l'eau. La solution aqueuse est ensuite lyophilisée (0,98g). Les fonctions méthyl-ester ainsi introduites sont caractérisées en infrarouge par une bande de vibration à 1740 $cm^{-1}$.

Stade 2

Le polymère obtenu au stade 1 est saponifié par la soude, et, après neutralisation par une résine de marque commerciale Dowex AG 50 W X 8, on obtient un polymère substitué par 75 groupements carboxyliques avec un rendement de 81% après lyophilisation.

Stade 3

Le polymère obtenu au stade 2 (400 mg, soit sensiblement 2 micromoles) est mis en solution dans le N, N diméthylformamide (2ml) en présence de Gly-Gly-L-Tyr-Daunorubicine, HCl (34 mg, 40 micromoles) et de triéthylamine (28 microlitres, soit 200 micromoles). Après addition d'hydroxybenzotriazole (7,7 mg, 50 micromoles) et de dicyclohexyl carbodiimide (10,3 mg, 50 micromoles), le mélange réactionnel est agité pendant 48 heures à température ambiante à l'abri de la lumière. A ce stade les fonctions carboxyliques résiduelles sont modifiées sous forme d'amide par addition de tris (hydroxyméthyl) aminoéthane (24 mg, 200 micromoles) en présence d'hydroxybenzotriazole (30,6mg) et de dicyclohexyl carbodiimide (41,2 mg). Après dilution, dialyse contre de l'eau et centrifugation du résidu insoluble, le polymère est lyophilisé (392 mg). La mesure de l'absorption à 495 nm permet de préciser qu'une molécule de polymère contient en moyenne 15 résidus de Gly-Gly-L-Tyr-Daunorubicine. Le rendement de fixation est de 73%.

EXEMPLE 2 :

Préparation d'un polymère linéaire de N-acryloylamino-2-hydroxyméthyl-2-propanediol-1,3 polycarboxylé substitué par la séquence -Gly-Gly-L-Tyr-L-Leu-Daunorubicine.

Le polymère obtenu au stade 2 de l'exemple 1 est couplé, dans les conditions utilisées au stade 3 de l'exemple 1, pour la séquence -Gly-Gly-L-Tyr-L-Leu-Daunorubicine, hormis l'utilisation de 18 équivalents de ce dérivé, étant donné le caractère hydrophobe de la séquence peptidique. Ainsi, 13 résidus de peptidyl Daunorubicine ont été fixés par molécule de polymère avec un rendement de 69%.

De façon analogue, on a préparé le même polymère, mais marqué au $^{14}C$ sur la daunorubicine en position 14.

EXEMPLE 3 :

Préparation d'un polymère linéaire de N-acryloylamino-2-hydroxyméthyl-2-propanédiol-1,3 polycarboxylé, substitué par la séquence Gly-L-Phé-L-Ala-L-Leu-Daunorubicine.

On opère comme décrit dans l'exemple 1 au stade 3, 16 équivalents du composé Gly-L-Phé-L-Ala-L-Leu-Daunorubicine étant utilisés pour la réaction. Dans ces conditions, 11 résidus de peptidyl-Daunorubicine sont couplés par molécule de polymère avec un rendement de 70%.

EXEMPLE 4 :

Préparation d'un polymère linéaire polygluconoyle polylysine polycarboxylé substitué par la séquence Gly-Gly-L-Tyr-L-Leu-Daunorubicine.

Stade 1

De la poly-L-lysine commerciale (origine BACHEM-500 mg) de masse moléculaire moyenne 50 000, est mise en solution dans le système-solvant N,N-diméthylformamide/diméthylsulfoxyde (3 : 1) (20 ml) puis déprotonée par addition de triéthylamine (335 microlitres). Cette solution est portée à 50°C et on ajoute toutes les 6 heures de la glucono-delta-lactone pendant 24 heures (4 fois 425 mg). Après refroidissement, le mélange réactionnel est dilué par l'eau (40 ml) dialysé, puis lyophilisé (470 mg). Après dosage, la poly-L-lysine ainsi modifiée ne possède plus de fonction amine libre.

Stade 2

Le polymère polygluconoyle polylysine préparé ci-dessus est modifié selon le protocole décrit dans les stades 1 et 2 de l'exemple 1, et mène à un polymère substitué par 20 groupements carboxyliques.

Stade 3

Le polymère obtenu au stade 2 de l'exemple 4 (470 mg 9 micromoles) est mis en solution dans le N,N diméthylformamide en présence de Gly-Gly-L-Tyr-L-Leu-Daunorubicine (86 mg 90 micromoles) et de triéthylamine (28 microlitres, soit sensiblement 200 micromoles). Après addition d'hydroxybenzotriazole (14,4 mg, 0,1 millimole) et de dicyclohexyl carbodiimide (20,6 mg, 0,1 millimole) le mélange réactionnel est agité 48 heures à température ambiante à l'abri de la lumière. Les fonctions carboxyliques résiduelles sont substituées sous forme d'amide par addition de tris(hydroxyméthyl)aminométhane. Après dilution et dialyse contre de l'eau, le polymère est lyophilisé (460 mg). Par mesure de l'absorption à 495 nm, 9 résidus de Gly-Gly-L-Tyr-L-Leu-Daunorubicine par molécule de polymère sont calculés. Le rendement de fixation est de 75%.

EXEMPLE 5 :

Préparation d'un poly-L-glutamate polyhydroxylé conjugué à la séquence Gly-Gly-L-Tyr-L-Leu-Daunorubicine.

Stade 1

De l'acide poly-L-glutamique commercial (origine BACHEM 500 mg, $10^{-2}$ mmoles) de masse moléculaire moyenne 50 000, est mis en solution dans du N,N-diméthyl-formamide (40 ml) anhydre en présence de triéthylamine (550 microlitres, 3,9 mmoles) et de Gly-Gly-L-Tyr-L-Leu-Daunorubicine (86 mg, 90 micromoles). Après addition d'hydroxybenzotriazole (14,4 mg, 0,1 mmole) et de dicyclohexyl carbodiimide (20,6 mg, 0,1 mmole), le mélange réactionnel est agité 48 heures à température ambiante à l'abri de la lumière. Le mélange

réactionnel est dilué dans un tampon phosphate, après une heure à 4°C, le matériel insoluble est éliminé par centrifugation, puis dialysé contre de l'eau et enfin lyophilisé. Par mesure de l'absorption à 495 nm, 8 molécules de Gly-Gly-L-Tyr-L-Leu-Daunorubicine par molécule de polymère sont appréciées.

Stade 2

Le polymère isolé au stade 1 (300 mg, 5,2 micromoles) est repris dans le N,N diméthylformamide (30 ml) en présence de tris(hydroxyméthyl)-aminométhane (242 mg, 2 mmoles). Après addition d'hydroxybenzotriazole (283 mg, 2,1 mmoles) et de dicyclohexyl carbodiimide (432 mg, 2,1 mmoles) le mélange réactionnel est agité 48 heures à température ambiante. Le polymère ainsi substitué est dialysé contre de l'eau, le résidu solide est éliminé par centrifugation, et le surnageant est lyophilisé.

De cette façon, toutes les fonctions carboxyliques présentes sur le polymère sont modifiées quantitativement sous forme d'amide du tris(hydroxyméthyl)aminométhane. L'absence des fonctions carboxyliques est montrée par dosage pH-métrique.

Stade 3

Le polymère isolé au stade 1 (200mg, 3,5 micromoles) est repris dans le N,N-diméthylformamide (7ml) en présence de N-méthylglucamine (454mg, 2,3 mmoles). Après addition d'hydrobenzotriazole (356mg, 2,3 mmoles) et de dicyclohexylcarbodiimide (479mg, 2,3 mmoles), le mélange réactionnel est agité une heure à 0°C puis 16 heures à température ambiante. Le polymère ainsi substitué est isolé par précipitation et centrifugation dans le système-solvant n-butanol/acétate d'éthyle (1:1, v/v), puis dialysé plusieurs fois contre de l'eau pendant 48heures. Le résidu insoluble éventuel est éliminé par centrifugation, et le surnageant est lyophilisé.

Dans ces conditions, 85% des fonctions carboxyliques sont modifiées sous forme d'amide de la N-méthylglucamine.

EXEMPLE 6

Préparation d'un polymère linéaire de N-acryloylamino-2-hydroxyméthyl-2-propanediol-1,3 polycarboxylé, substitué par la séquence Gly-L-Pro-L-Arg-L-Leu-Daunorubicine.

On opère comme décrit dans l'exemple 1 au stade 3, 20 équivalents du composé Gly-L-Pro-L-Arg-L-Leu-Daunorubicine étant utilisés pour la réaction. Dans ces conditions, 13 résidus de peptidyl-Daunorubicine sont couplés par molécule de polymère avec un rendement de 65%.

EXEMPLE 7

Protéolyse *in vitro*

L'hydrolyse chymotrypsique est réalisée dans un tampon Tris-HCl 0,05M, $CaCl_2$ 10mM à pH 8,0.

On analyse les dérivés de la daunorubicine libérés après hydrolyse enzymatique, en chromatographie sur couche mince. Par exemple :

- le polymère de l'exemple 2 libère la leucyl-Daunorubicine : Rf : 0,4 dans le système-solvant $CHCl_3/CH_3OH$ (9 : 2).

- le polymère de l'exemple 1 libère la Daunorubicine : Rf 0,56 dans le système-solvant $CHCl_3/CH_3OH/H_2O$/acide acétique (15:5:1:1).

- Le polymère de l'exemple 3 libère l'alaninyl-leucyl-Daunorubicine: Rf 0,32 dans le système-solvant $CHCl_3/CH_3OH$ (9:2).

L'hydrolyse trypsique est réalisée dans une solution de bicarbonate de sodium 0,1 M, $CaCl_2$ 10 mM à pH 8,7.

Le polymère de l'exemple 6 est hydrolysé par la trypsine en libérant la leucyl-Daunorubicine : Rf 0,4 dans le système-solvant $CHCl_3/CH_3OH$ (9:2).

PROPRIETES PHARMACOLOGIQUES

1) Les cellules du carcinome pulmonaire de Lewis (3LL) proviennent d'une tumeur maligne métastasante, se développant spontanément chez les souris C 57 BL/6 Mayo J.C. (1972) Cancer Chem. Rep. Vol.3, 325.

a) Culture *in vitro*

Ces cellules sont cultivées en monocouches (ensemencées à 2 x $10^4$ cellules/ml) dans un milieu MEM contenant 20% de sérum de veau foetal à 37°C et sous atmosphère de 5% $CO_2$ - 95% air. A confluence (après 3 jours) elles sont récupérées par un bref traitement (2 minutes) par une solution d'EDTA 0,02% dans du PBS 0,1% glucose. Les cellules sont lavées avec un milieu MEM, puis reprises dans le milieu complet. Viabilité >95%.

b) Tumeur solide *in vivo*

L'obtention de cellules isolées à partir d'une tumeur solide nécessite l'utilisation de protéases et d'agents chélatants. La tumeur est prélevée et débarrassée des zones nécrosées. Elle est ensuite découpée en petits fragments d'environ 1 $mm^3$ et mise en suspension dans du PBS contenant 0,1% de glucose, pendant 10 minutes sous agitation à 4°C. Le surnageant est éliminé et les fragments sont mis en présence de trypsine (Trypsine Enzar T pour culture cellulaire, Reheis Chemical Company, Armour Pharmaceutical, South Plainfield, USA), et 0,02% d'EDTA (Merck) dans le PBS glucosé, à la température ambiante. Les cellules se détachent peu à peu et sont collectées, après 30 minutes dans un tube à centrifuger après filtration sur toile à bluter de nylon (Monyl Ny25 HC, mailles de 25 micromètres, Polylabo, Strasbourg, France). Après deux lavages dans du PBS glucosé, les cellules vivantes sont comptées et leur concentration ajustée.

JT/AA - BR.73674

2) Activité cytotoxique in vitro

L'activité cytotoxique est mesurée par l'inhibition de l'incorporation de la thymidine tritiée.

Dans les puits d'une plaque Limbro, on introduit 1 ml d'une suspension cellulaire de cellules 3 LL à $10^4$ cellules/ml dans un milieu MEM contenant 20% de sérum de veau foetal. Les cellules sont ainsi mises en culture pendant 24 heures. Le milieu de culture est éliminé par aspiration et on dispose ensuite dans chaque puits, 1 ml de diverses dilutions de drogue réalisées avec le milieu de culture. Les plaques sont disposées dans l'étuve durant 43 heures. On additionne ensuite 20 microlitres d'une solution de thymidine tritiée (0,5 microCurie, 18,5 KBq). Après 4 heures d'incubation les cellules sont décollées avec une raclette de sécurité, puis récupérées sur filtre en fibre de verre et lavées. On mesure la radioactivité des cellules dans un compteur à scintillations bêta après dissolution dans un liquide scintillant.

L'activité cytotoxique des drogues et conjugués suivants a été déterminée sur les cellules 3 LL selon le protocole décrit ci-dessus :

- Daunorubicine libre
- leucyl-Daunorubicine
- Gly-Gly-L-Tyr-L-Leu-Daunorubicine
- Polymère de l'exemple 1
- Polymère de l'exemple 2
- Polymère de l'exemple 3

Sur la base des résultats obtenus en incorporation de $^3H$ thymidine réunis dans la figure 1, on voit que la substitution de la Daunorubicine par un peptide, ou mieux, son greffage sur un polymère linéaire, entraîne une diminution plus importante de la cytotoxicité de cette drogue in vitro. L'activité cytotoxique est modulée selon la séquence peptidique.

3) Expériences in vivo

Afin d'étudier la distribution d'un polymère macromoléculaire dans l'organisme de la souris, après injection intraveineuse, ou intrapéritonéale, et sa durée de vie plasmatique, on utilise le polymère marqué au $^{14}C$ sur la Daunorubicine en position 14 préparé à l'exemple 2.

a) Clairance plasmatique

Le polymère marqué au $^{14}C$ en solution dans du sérum physiologique à la concentration 15 mg/ml est injecté (200 microlitres, 3 mg de polymère correspondant à 100 microgrammes de Daunorubicine), soit par voie intraveineuse dans une veine latérale de la queue, soit par injection intrapéritonéale.

La clairance de ce polymère dans le flux sanguin est mesurée par prélèvement de 50 microlitres de sang à divers temps après l'injection. Le sang est prélevé dans le plexus veineux de l'oeil à l'aide d'une micropipette calibrée prétraitée avec un anticoagulant. L'activité présente dans chaque échantillon est comptée après dilution dans un liquide scintillant (2,5 ml).

Les résultats sont représentés à la figure 2 et sont résumés ci-après :

- La décroissance de la concentration plasmatique du polymère est relativement faible, en effet au bout de 48 heures on retrouve 20% de la radioactivité initiale. Ce résultat tout à fait remarquable, compte tenu du fait que l'adriamycine disparaît du plasma en 20 minutes.

- L'analyse chromatographique sur couche mince révèle en outre que le matériel radioactif dans le plasma se trouve toujours sous une forme macromoléculaire. Le tétrapeptide intercalé entre le polymère et la drogue est donc relativement résistant aux protéases sériques.

- La drogue macromoléculaire peut passer de la cavité péritonéale dans la circulation sanguine. Trois heures après l'injection, le taux plasmatique du polymère injecté par voie intrapéritonéale est semblable à celui du polymère injecté par voie intraveineuse. L'analyse chromatographique sur couche mince de silice montre ici encore que le matériel radioactif dans le plasma se trouve encore sous forme macromoléculaire. Mais des injection intrapéritonéales répétées peuvent ralentir le transfert de la macromolécule de la cavité péritonéale vers le flux sanguin.

b) Localisation

Les drogues radiomarquées sur la (14-$^{14}$C) Daunorubicine sont injectées par voie intraveineuse 14 jours après l'implantation de $10^5$ cellules 3 LL. On injecte soit 100 microgrammes de Daunorubicine libre (5 x $10^5$ c.p.m.); soit 100 microgrammes de Daunorubicine sous forme macromoléculaire (5 x $10^5$ c.p.m.) ; la localisation est mesurée après l'injection intraveineuse des différents produits.

Les souris sont sacrifiées par élongation cervicale 48 heures ou 72 heures après l'injection. Les organes sont prélevés, dissous par un agent solubilisant de tissus (NCS Amersham) à raison de 200 mg à 300 mg d'organe/ml de solubilisant, puis comptés dans un liquide scintillant. Les expériences sont réalisées sur des lots de 3 souris avec le polymère de l'exemple 2 et la Daunorubicine libre. Les résultats sont réunis dans la figure 3.

Une persistance générale de la Daunorubicine dans l'organisme est observée lorsque celle-ci se trouve sous forme macromoléculaire, notamment au niveau de la tumeur.

L'examen des organes au moment de leur prélèvement, puis de leur pesée montre une atrophie sévère de la rate sous l'effet de la Daunorubicine libre. Cette atrophie coïncide avec une accumulation importante de la radioactivité, alors que les rates des souris traitées avec le polymère peptidyl drogue ont une faible radioactivité et une masse comparable à celle des animaux sains.

c) Activité antitumorale

L'activité antitumorale in vivo des prodrogues macromoléculaires est évaluée sur des souris mâles C 57 BL/6 agées d'une dizaine de semaines. On implante $10^5$ cellules 3 LL à chaque souris par injection intramusculaire de 100 microlitres d'une suspension cellulaire à $10^6$ cellules/ml dans la patte postérieure droite.

- Un lot de 10 souris servant de témoins est injecté avec 7 x 100 microlitres de sérum physiologique.
- Un lot de 10 souris est traité avec 7 x 100 microgrammes de Daunorubicine libre (dose limite due à la toxicité de la drogue libre).
- Un lot de 10 souris est traité avec le polymère de l'exemple 2 à raison de 8 x 200 microgrammes équivalents en Daunorubicine.

Après injection intramusculaire des cellules tumorales, la prodrogue macromoléculaire est injectée par voie intraveineuse aux jours 2, 6 et 10 puis par voie intrapéritonéale aux jours 14, 18, 22, 26 et 30.

Deux paramètres sont enregistrés pour évaluer l'activité antitumorale in vivo : l'inhibition de croissance de la tumeur et la survie.

L'examen de la courbe de croissance (figure 4) de la tumeur montre que la Daunorubicine même à la dose employée (dose maximale eu égard à sa toxicité) n'influe pas de façon significative sur la croissance tumorale. Le polymère de l'exemple 2 par contre montre une inhibition de croissance significative en chaque point par rapport au lot témoin.

Le second paramètre enregistré, survie des animaux traités par rapport aux animaux témoins, confirme l'activité antitumorale du polymère de l'exemple 2 (figure 5). Le T/C mesuré est de 145.

Il est à noter que la fixation de la Daunorubicine sur le polymère par l'intermédiaire d'un peptide diminue notamment sa toxicité secondaire, ce qui permet l'emploi de doses importantes en équivalent de Daunorubicine.

REVENDICATIONS

1. Prodrogues caractérisées par le fait qu'elles comprennent essentiellement une drogue ou substance active liée par une liaison covalente à un polymère polyhydroxylé par l'intermédiaire d'un groupement peptidyle, ledit polymère polyhydroxylé étant un polymère dont au moins 20% des motifs sont des motifs polyhydroxylés.

2. Prodrogues selon la revendication 1, caractérisées par le fait quele rapport du nombre moyen de molécules de substance active fixée sur une molécule de polymère au nombre moyen de groupements hydroxyle présents sur une molécule du polymère peut varier de $10^{-2}$ à $10^{-4}$ .

3. Prodrogues macromoléculaires caractérisées par le fait qu'elles répondent à la formule I

$$M-(P-D)_n \qquad (I)$$

dans laquelle M est le reste d'une molécule polymérique dont au moins 20% des motifs sont des motifs polyhydroxylés, P est un groupement peptidyle, D est le reste de la molécule de drogue et n représente le nombre de molécules de drogue D fixées sur la molécule polymérique M.

4. Prodrogues selon l'une quelconque des revendications précédentes, caractérisées par le fait que ladite drogue est une drogue aminée liée au groupement peptidyle P par l'extrémité C-terminale de celui-ci, et dans laquelle le groupement peptidyle est lié à la molécule polymérique par l'intermédiaire d'une liaison covalente.

5. Prodrogues selon la revendication 4, caractérisées par le fait que ladite liaison covalente entre la molécule M et le peptide P est une liaison amide.

6. Prodrogues selon l'une quelconque des revendications précédentes, caractérisées par le fait que ledit polymère est un polymère d'acrylamide substitué à l'azote par un groupement hydrocarboné polyhydroxylé.

7. Prodrogues selon l'une quelconque des revendications 1 à 5, caractérisées par le fait que ledit polymère est un homopolymère d'aminoacide diaminé dont le groupement aminé non impliqué dans la liaison polymérique est substitué par un groupement polyhydroxyalcanoyle.

8. Prodrogues selon la revendication 7, caractérisées par le fait que ledit homopolymère est un polymère à base de polylysine dont les groupements $-NH_2$ libres sont acylés par un agent d'acylation dérivé d'un acide polyhydroxyalcanoïque.

9. Prodrogues selon l'une quelconque des revendications 1 à 5, caractérisées par le fait que ledit polymère est un homopolymère d'acide aminé dicarboxylique dont une partie des groupements acide carboxylique non impliqués dans la liaison polymérique sont amidifiés par une polyhydroxy-alkylamine.

10. Prodrogues selon la revendication 3, caractérisées par le fait que M représente le reste d'un polymère IA constitué essentiellement de motifs de formules :

$-CH(R^1)-C(R^2)(X^1)-$ et $-CH(R^1)-C(R^2)(X^2)-$

(IIA) (IIIA)

dans lesquelles :

$R_1$ et $R_2$ représentent -H ou un groupement alkyle inférieur,

$X^1$ représente un groupement-CO-NH-$R^3$,

$X^2$ représente un groupement-CO-NH-X-CO-P-D,

$R_3$ est un groupement hydrocarboné polyhydroxylé,

X représente un groupement hydrocarboné ayant 1 à 8 atomes de carbone, éventuellement interrompu par 1 ou plusieurs hétéroatomes d'oxygène de soufre ou d'azote, et P et D sont définis comme précédement ;

11. Prodrogues selon la revendication 3, caractérisées par le fait que M représente le reste d'un polymère IB constitué essentiellement de motifs de formules :

$-CO-CH(X^3)-NH-$ et $-CO-CH(X^4)-NH-$

(IIB) (IIIB)

dans lesquelles :

$X^3$ représente un groupement $-(CH_2)_x-NH-CO-R^4$,

$X^4$ représente un groupement $-(CH_2)_x-NH-CO-P-D$,

x est un nombre entier pouvant varier de 1 à 4, $R_4$ est un groupement hydrocarboné polyhydroxylé, et P et D sont définis comme précédemment ;

12. Prodrogues selon la revendication 3, caractérisées par le fait que M représente le reste d'un polymère IC constitué essentiellement de motifs de formules:

$-CO-CH(X^5)-NH-$ et $-CO-CH(X^6)-NH-$

(IIC) (IIIC)

dans lesquelles :

$X^5$ représente un groupement $-(CH_2)_y-CO-NH-R^5$,

$X^6$ représente un groupement $-(CH_2)_y-CO-P-D$,

y est un nombre entier pouvant varier de 1 à 4

$R_5$ est un groupement hydrocarboné polyhydroxylé,

et P et D sont définis comme précédemment.

13. Prodrogues selon l'une quelconque des revendications précédentes, caractérisées par le fait que le groupement peptidyle comporte l'une des séquences énumérées ci-après, étant entendu qu'il est possible d'insérer 1 à 3 autres acides aminés entre la molécule polymérique et la séquence peptidique indiquée :

-L-Ala-L-Ala-L-Pro-L-Ala-D
-L-Ala-L-Ala-L-Pro-L-Ala-L-Leu-D
-L-Ala-L-Ala-L-Pro-L-Ala-D
-L-Ala-L-Ala-L-Pro-L-Ala-L-Leu-D
-L-Ala-L-Ala-L-Pro-L-Val-D
-L-Ala-L-Ala-L-Pro-L-Val-L-Leu-D
-L-Ala-L-Ala-L-Pro-L-Val- D
-L-Ala-L-Ala-L-Pro-L-Val-L-Leu-D
-L-Phé-L-Val-L-Arg-D
-L-Phé-L-Val-L-Arg-L-Leu-D
-L-Phé-L-Val-L-Arg-D
-L-Phé-L-Val-L-Arg-L-Leu-D
-L-Phé-L-Ala-L-Lys-D
-L-Phé-L-Ala-L-Lys-L-Leu-D
-L-Phé-L-Ala-L-Lys-D
-L-Phé-L-Ala-L-Lys-L-Leu-D
-Gly-Gly-L-Arg-D
-Gly-Gly-L-Arg-L-Leu-D
-Gly-Gly-L-Arg-D
-Gly-Gly-L-Arg-L-Leu-D
-Gly-Gly-L-Tyr-D
-Gly-Gly-L-Tyr-L-Leu-D
-Gly-Gly-L-Tyr-D
-Gly-L-Phé-L-Ala-L-Leu-D
-Gly-L-Pro-L-Arg-L-Leu-D
-L-Pro-L-Phé-L-Arg-D

-<u>L</u>-Pro-<u>L</u>-Phé-<u>L</u>-Arg-<u>L</u>-Leu-D

-<u>L</u>-Pro-<u>L</u>-Phé-<u>L</u>-Arg-D

-<u>L</u>-Pro-<u>L</u>-Phé-<u>L</u>-Arg-<u>L</u>-Leu-D

-<u>L</u>-Val-<u>L</u>-Leu-Gly-<u>L</u>-Arg-D

14. Prodrogues selon l'une quelconque des revendications précédentes, caractérisées par le fait que ladite drogue D est une drogue antitumorale, antibactérienne ou antiparasitaire.

15. Prodrogues selon la revendication 14, caractérisées par le fait que ladite drogue est choisie parmi la daunorubicine, l'adriamycine, la puromycine, la primaquine et la chloroquine.

16. Utilisation, dans la réalisation d'une prodrogue, d'un polymère polyhydroxylé auquel la drogue est reliée par l'intermédiaire d'un groupement peptidyle, ledit polymère ayant au moins 20% de motifs polyhydroxylés.

17. Utilisation selon la revendication 16, caractérisée par le fait que ledit polymère polyhydroxylé est tel que défini dans l'une quelconque des revencications 6 à 12.

18. Procédé de préparation d'une prodrogue telle que définie dans l'une quelconque des revendications précédentes, caractérisé par le fait :

a) soit que l'on utilise comme produit de départ une macromolécule contenant des motifs polyhydroxylés que l'on éthérifie avec un groupement hydrocarboné comportant un groupement carboxylique protégé, que l'on soumet l'éther obtenu à une réaction de déprotection du groupe carboxylique et que l'on fait agir le composé obtenu avec un composé peptidyl-drogue tel qu'un composé de formule :

$$H - P - D$$

dans laquelle P et D sont définis comme précédemment ;

b) soit que l'on utilise comme produit de départ une macromolécule polycarboxylée dont le groupement carboxylique est activé et que l'on fait réagir lesdits groupements carboxyliques activés partiellement avec un composé peptidyl-drogue tel qu'un composé de formule :

$$H - P - D$$

P étant défini comme précédemment, et partiellement avec une amine primaire ou secondaire polyhydroxylée telle qu'une amine de formule:

$$R_5-NH_2$$

$R_5$ étant défini comme précédemment ;

c) soit que l'on utilise comme produit de départ une macromolécule polyaminée que l'on fait réagir avec un agent d'acylation dérivé d'un acide carboxylique polyhydroxylé pour obtenir, par réaction d'amidification, une molécule polyhydroxylée sur laquelle on poursuit la synthèse comme en a) ci-dessus.

JT/AA - BR.73674

19. Utilisation des prodrogues telles que définies dans l'une quelconque des revendications 1 à 15, dans la préparation industrielle de médicaments.

20. Médicament caractérisé par le fait qu'il comprend comme ingrédient actif au moins une prodrogue telle que définie dans l'une quelconque des revendications 1 à 15.

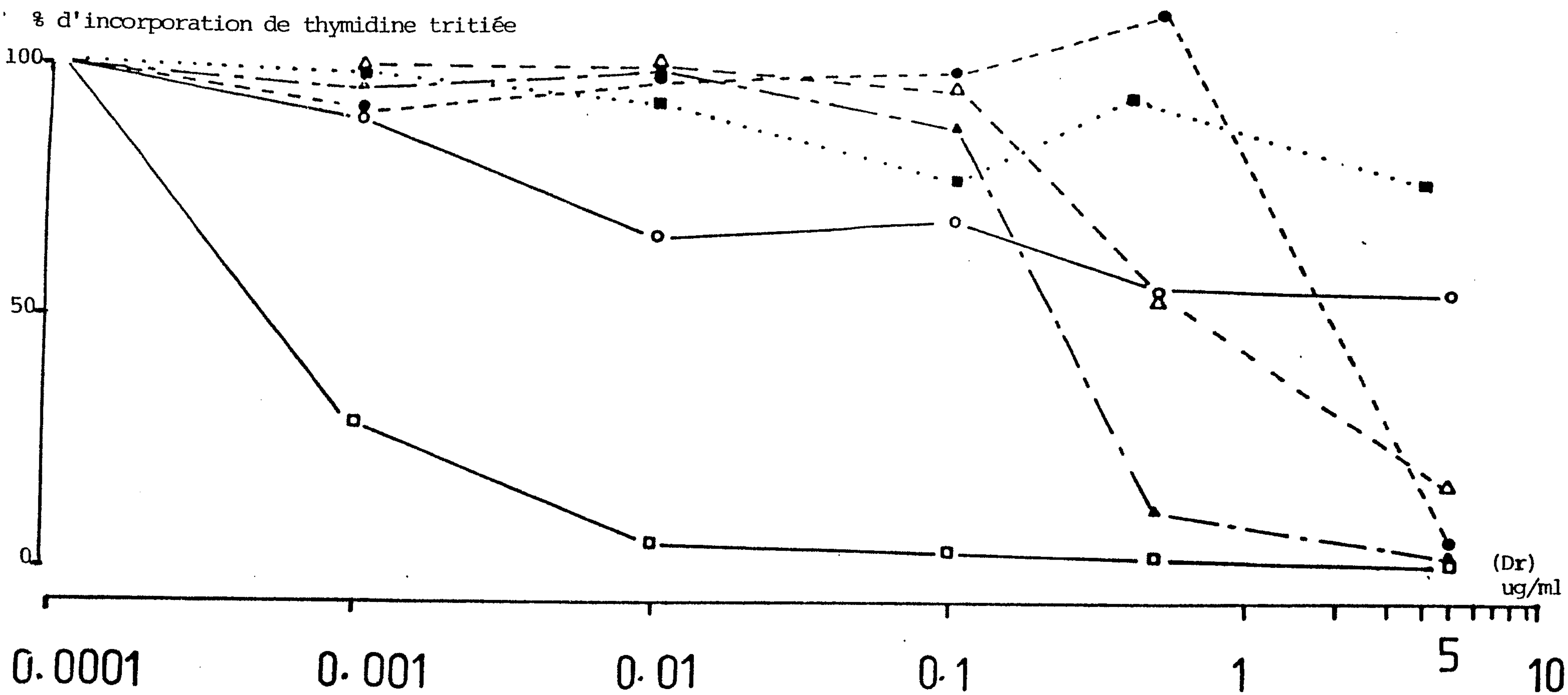


Figure 1 : Mesure de l'activité cytotoxique <u>in vitro</u> sur des cellules 3 LL.

□—□ Daunorubicine libre,

▲-·-▲ leucyl-Daunorubicine

△-·-△ Gly-Gly-L-Tyr-L-Leu-Daunorubicine

●--● Polymère de l'exemple 1

○—○ Polymère de l'exemple 2

■····■ Polymère de l'exemple 3

0208576

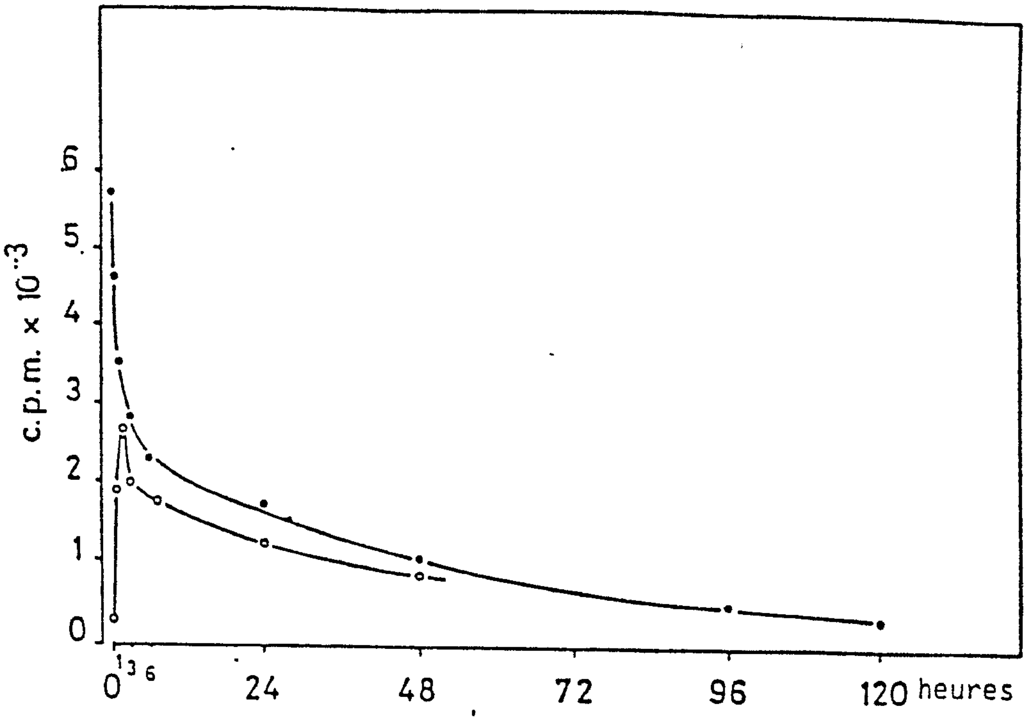


Figure 2 : Elimination plasmatique du polymère de l'exemple 2 marqué au $^{14}C$ ;

—— ● —— : injection intraveineuse ($4 \times 10^5$ c.p.m.),

—— O —— : injection intrapéritonéale ($4 \times 10^5$ c.p.m.)

c.p.m. : coups par minute.

0208576

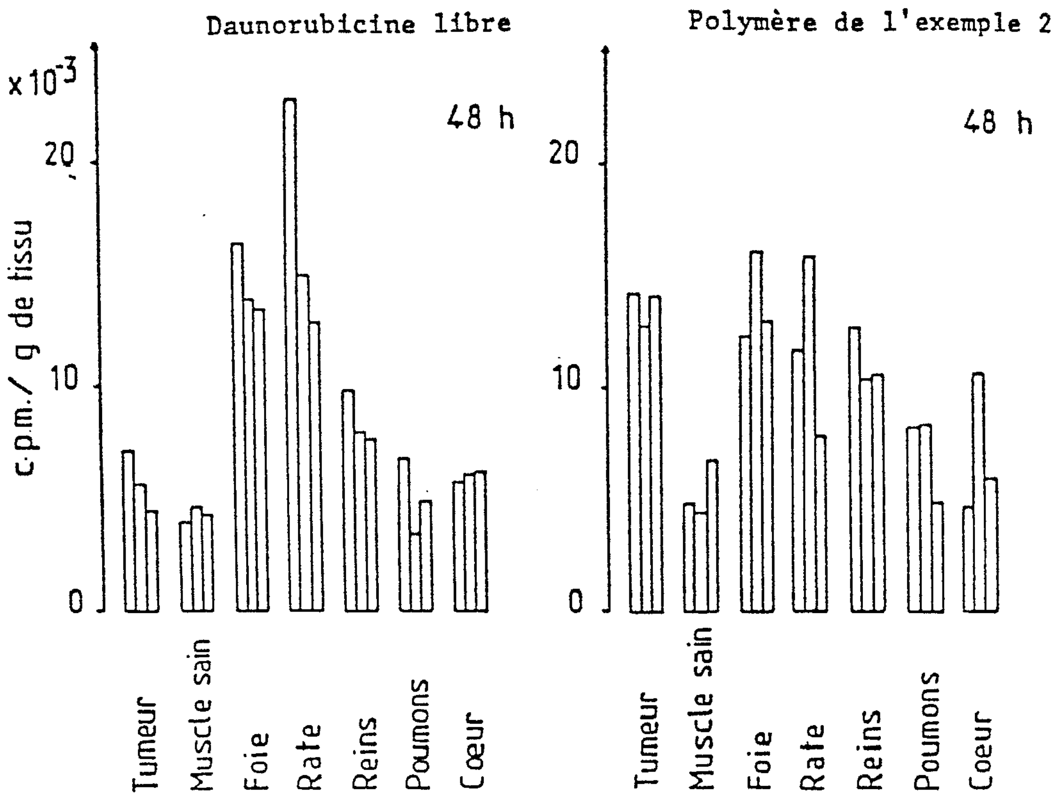


Figure 3 : Localisation de la (14 - $^{14}$C) Daunorubicine soit libre, soit sous la forme du polymère de l'exemple 2 chez des souris C 57 Bl/6 porteuses d'un carcinome pulmonaire de Lewis.

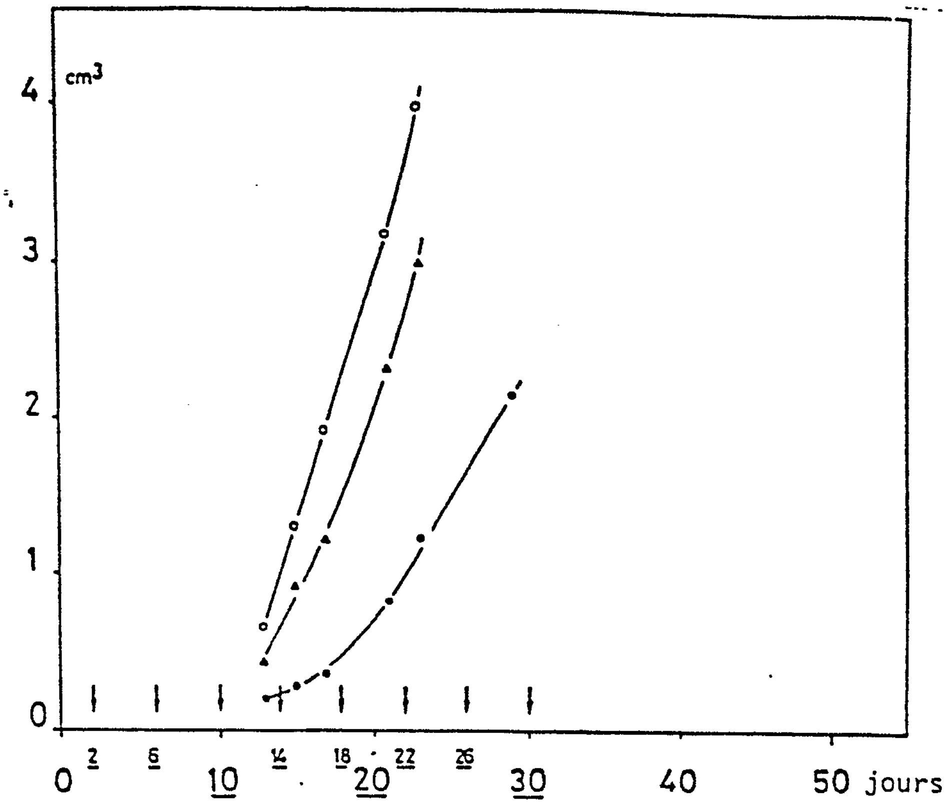


Figure 4 : Mesure de la croissance tumorale en fonction du temps ↓ : jours d'injection des différents produits, o : sérum physiologique, ▲ : Daunorubicine libre (100 microgrammes/injection), ● : Polymère de l'exemple 2 (200 microgrammes/injection en équivalents de Daunorubicine).

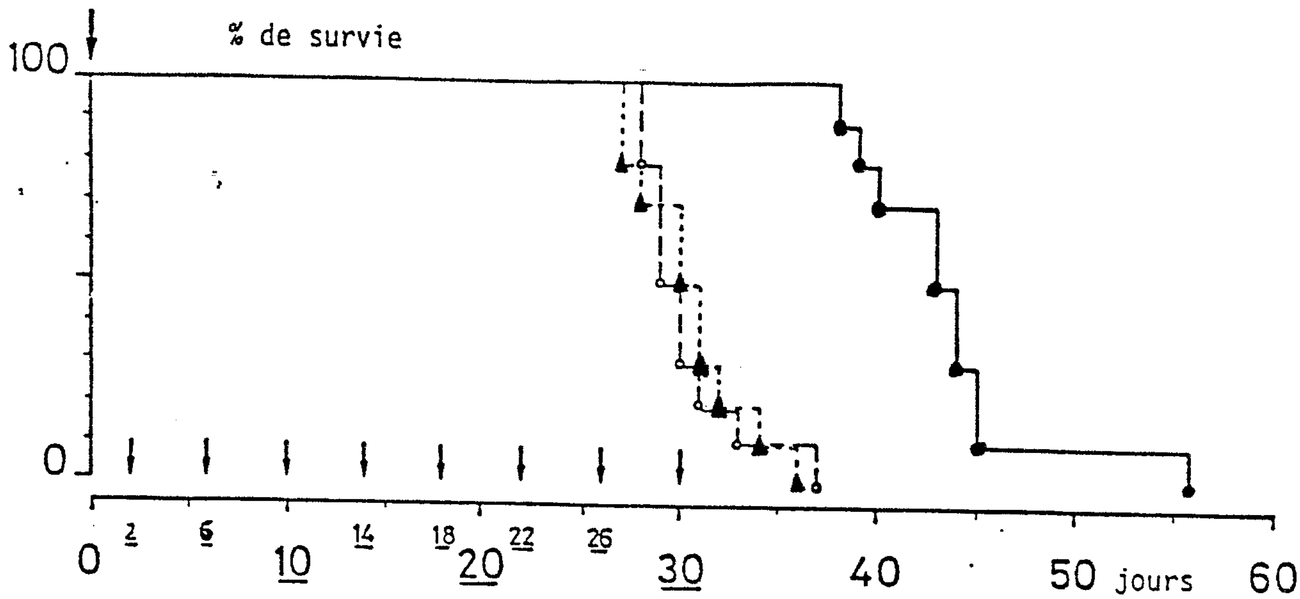


Figure 5 : Mesure de la survie des animaux traités ; Jo : implantation intramusculaire de $10^5$ cellules 3 LL, ↓ : jours d'injection des différents produits, o--o--o : sérum physiologique, ▲-▲--▲ : Daunorubicine libre (100 microgrammes injection, ●--●--● : Polymère de l'exemple 2 (200 microgrammes/injection en équivalents de Daunorubicine).

Office européen des brevets

**0208576**

Numero de la demande

## RAPPORT DE RECHERCHE EUROPEENNE

EP 86 40 1227

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int. Cl.4) |
| X | EP-A-0 037 388 (INSTITUT INTERNATIONAL DE PATHOLOGIE CELLULAIRE ET MOLECULAIRE)<br>* Revendications 1-3,6,13 * | 1,4,5, 14-20 | A 61 K 47/00<br>A 61 K 31/765 |
| Y | | 13 | |
| | --- | | |
| X | ADVANCES ON POLYMER SCIENCE, vol. 57, 1984, pages 51-101, Springer Verlag, Berlin, DE; K. DUSEK et al.: "Polymers in medicine"<br>* Page 61, lignes 22-28; page 67, paragraphe 3; page 69, paragraphe 3 - page 70, paragraphe 4; page 75, paragraphe 2; page 79, paragraphe 4 * | 1,4-6, 10 | |
| | | | DOMAINES TECHNIQUES RECHERCHES (Int. Cl.4) |
| Y | IDEM<br>* Page 63, paragraphe 3 * | 13 | A 61 K |
| | --- | | |
| X | GB-A-1 541 436 (G.D. SEARLE & CO.)<br>* Revendications 1,5,8 * | 1-9 | |
| | ----- | | |

Le présent rapport de recherche a été établi pour toutes les revendications

| Lieu de la recherche | Date d'achèvement de la recherche | Examinateur |
|---|---|---|
| LA HAYE | 25-09-1986 | TZSCHOPPE,D.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82